# EUROPEAN PATENT SPECIFICATION

(11) **EP 4 294 307 B1**
(45) Date of publication and mention of the grant of the patent: **09.12.2026**
(21) Application number: 22720809.7
(22) Date of filing: 25.03.2022
(51) Int. Cl.: A61B 34/00, A61B 90/00

(54) **MEDICAL DEVICE PROJECTION SYSTEM**
PROJEKTIONSSYSTEM FÜR MEDIZINISCHE VORRICHTUNG
SYSTÈME DE PROJECTION DE DISPOSITIF MÉDICAL

(30) Priority: 26.03.2021 US 202163166825 P
(43) Date of publication of application: 27.12.2023
(73) Proprietor: C. R. Bard, Inc., Franklin Lakes, NJ 07417 (US)
(72) Inventor: FRANKLIN, Jeff, E., Hamilton, OH 45011 (US); MANNION, Paul, T., Eliot, ME 03903 (US); IVLEV, Alex, Milcreek, UT 84107 (US)
(74) Representative: Hoffmann Eitle
(86) International application number: PCT/US2022/021936
(87) International publication number: WO 2022/204506

(56) References cited:
- WO-A1-2016/133644
- WO-A1-2018/226850
- WO-A1-2019/236505
- US-A1- 2019 125 454

## Description

### PRIORITY

This application claims the benefit of priority to U.S. Provisional Application No. 63/166,825, filed March 26, 2021.

### BACKGROUND

When a sterile field is present during a medical procedure, it can be difficult for a clinician to interact with, provide input to or change parameters on a medical device used in the procedure when the medical device is located outside of the sterile field. The clinician must exit the sterile field or relay the clinician's input to a person outside of the sterile field. This process can take time away from the procedure and require reagents for sterilization of the clinician each time the clinician exits the sterile field. It would be beneficial to the clinician be able to interact with, provide input to or change parameters on the medical device while maintain sterility within the sterile field. Disclosed herein is a medical device projection system and a method that address the foregoing. WO 2019/236505 A1 relates to a medical device-placing system including a medical-device tip-location sensor configured for placement on a chest of a patient, an ultrasound probe, a console, and an alternative-reality headset.
WO 2016/133644 A1 relates to an augmented reality head mounted device worn by a user. US 2019/125454 A1 relates to a method for adjusting the operation of a clip applier using machine learning in a surgical suite.
WO 2018/226850 A1 relates to user interface systems for sterile fields and other working environments.

### SUMMARY

The present invention is defined by the medical device projection system according to independent claim 1 and the method according to independent claim 11. Other preferred features are recited in the dependent claims. Disclosed herein is a medical device projection system including a first medical device and a projector. The first medical device is configured to be outside of a sterile field and the first medical device includes a user control interface including a plurality of parameters. The projector is configured to be within the sterile field, and is in communication with the first medical device and is configured to project a mixed reality visual representation of the user control interface on a surface.

In some embodiments, the surface is visible to a user and is inside the sterile field or outside the sterile field.

In some embodiments, the surface includes a floor, a ceiling, a surface of a second medical device, or a sterile drape.

The projector includes one or more buttons configured to control the plurality of parameters, the one or more buttons being coupled to a left side, a right side, or a back side of a projector body.

In some embodiments, the one or more buttons include a clickable scroll wheel.

In some embodiments, the projector includes a projector body coupled to a projector stand, the projector stand coupled to a right side or a left side of the projector body.

In some embodiments, the projector stand includes a projector stand base or two or more projector stand legs.

In some embodiments, the projector is configured to rotate annularly around a projector stand axis.

In some embodiments, the projector includes a console having an energy source, one or more processors, non-transitory computer readable medium and a plurality of logic modules.

In some embodiments, the plurality of logic modules, when executed by the processor, are configured to perform operations including receiving a depiction of the user control interface from the first medical device, correlating the status of the one or more buttons with one or more parameter changes on the user control interface, projecting a mixed reality visual representation of the user control interface, including any parameter changes on a surface, and transmitting the one or more parameter changes from the projector to the first medical device.

In some embodiments, the first medical device includes a laser and the plurality of parameters include laser pulse energy, laser frequency, and laser pulse duration.

In some embodiments, the projector is configured to be sterile or placed into a sterile bag.

Also disclosed is a medical device projection system including a first medical device, a projector, and a display screen. The first medical device is configured to be outside of a sterile field, and includes a user control interface including a plurality of parameters. The projector is configured to be in communication with the first medical device, the projector configured to project a mixed reality visual representation of the user control interface including the plurality of parameters of the first medical device on a display screen being visible to a user and located within the sterile field.

In some embodiments, the display screen includes a heads up display screen in communication with the projector.

In some embodiments, the display screen includes one or more buttons configured to control the plurality of parameters, the one or more buttons configured to be coupled to a left side or a right side of the display screen.

In some embodiments, the one or more buttons include a clickable scroll wheel.

In some embodiments, the display screen is sterile or configured to be placed in a sterile bag.

In some embodiments, the projector is outside of the sterile field.

In some embodiments, the projector is inside of the sterile field and is sterile or configured to be placed in a sterile bag.

In some embodiments, the projector is coupled to the first medical device or to a second medical device, the second medical device inside the sterile field or outside of the sterile field.

In some embodiments, the display screen includes a display screen console having an energy source, one or more processors, non-transitory computer readable medium and two or more logic modules.

In some embodiments, the two or more logic modules, when executed by the processor, are configured to perform operations including correlating the statue of the one or more buttons with one or more parameter changes on the user control interface, and transmitting the one or more parameter changes to the projector.

In some embodiments, the projector includes a projector console having an energy source, one or more processors, non-transitory computer readable medium and a plurality of logic modules.

In some embodiments, the plurality of logic modules, when executed by the processor, are configured perform operations including receiving the depiction of the user control interface from the first medical device, projecting the mixed reality visual representation of the user control interface, including any parameter changes on the display screen, receiving the one or more parameter changes from the display screen, and transmitting the one or more parameter changes from the projector to the first medical device.

In some embodiments, the display screen is curved.

In some embodiments, the first medical device includes a laser, and the plurality of parameters include laser pulse energy, laser frequency or laser pulse duration.

Also disclosed is a method of controlling a plurality of parameter changes of a medical device from within a sterile field. The method includes placing a projector, having one or more buttons, in communication with a first medical device, the first medical device having a user control interface with a plurality of parameters, the first medical device being outside of a sterile field. The method further includes placing the projector in the sterile field, projecting a mixed reality visual representation of the user control interface on a surface, and providing one or more parameter changes to the first medical device.

In some embodiments, placing the projector having the one or more buttons in communication with the first medical device includes placing the projector having the one or more buttons configured to control the plurality of parameters, in communication with the first medical device.

In some embodiments, placing the projector in the sterile field includes placing the projector in a sterile bag.

In some embodiments, projecting the mixed reality visual representation of the user control interface on the surface includes projecting the mixed reality visual representation of the user control interface on the surface wherein the surface is visible to a user and includes a floor, a ceiling, a surface of a second medical device, or a sterile drape.

In some embodiments, providing one or more parameter changes to the first medical device include providing one or more parameter changes to the first medical device wherein the first medical device includes a laser and the one or more parameter changes include laser pulse energy, laser frequency or laser pulse duration.

Also disclosed is a method of controlling a plurality of parameter changes of a medical device from within a sterile field including placing a projector in communication with a first medical device, the first medical device having a user control interface with a plurality of parameters, the first medical device being outside of a sterile field. The method further includes placing a display screen having one or more buttons, in communication with the projector, placing the display screen in the sterile field, projecting a mixed reality visual representation of the user control interface on the display screen, and providing one or more parameter changes to the first medical device.

In some embodiments, placing the projector in communication with the first medical device, the first medical device having the user control interface with the plurality of parameters includes placing the projector in communication with the first medical device wherein the first medical device is a laser and the plurality of parameters include laser pulse energy, laser frequency, or laser pulse duration.

In some embodiments, placing the display screen in the sterile field includes placing the display screen in a sterile bag.

In some embodiments, providing one or more parameter changes to the first medical device include the one or more buttons of the display screen being manipulated by the user to provide the one or more parameter changes to the first medical device.

These and other features of the concepts provided herein will become more apparent to those of skill in the art in view of the accompanying drawings and following description, which describe particular embodiments of such concepts in greater detail.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 illustrates a perspective view of a medical device projection system, in accordance with some embodiments.
FIG. 2A illustrates a cross sectional side view of a projector of the medical device projection system, in accordance with some embodiments.
FIG. 2B illustrates a plan view of the projector, in accordance with some embodiments.
FIGS. 2C-2D illustrate a cross sectional side view of an exemplary method of adjusting the projector to project onto a variety of surfaces, in accordance with some embodiments
FIG. 3 illustrates a block diagram of some components of the medical device projection system including the projector and a projector console, in accordance with some embodiments.
FIG. 4 illustrates a perspective view of the medical device projector system including the projector and a display screen in accordance with some embodiments.
FIG. 5 illustrates a block diagram of some components of the medical device projection system including the projector console, a display screen, and a display screen console, in accordance with some embodiments.
FIG. 6 illustrates a flow chart of an exemplary method of controlling a plurality of parameter changes of a medical device from within a sterile field, in accordance with some embodiments.
FIG. 7 illustrates a flow chart of an exemplary method of controlling a plurality of parameter changes of a medical device from within a sterile field, in accordance with some embodiments

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

The term "logic" may be representative of hardware, firmware or software that is configured to perform one or more functions. As hardware, the term logic may refer to or include circuitry having data processing and/or storage functionality. Examples of such circuitry may include, but are not limited or restricted to a hardware processor (e.g., microprocessor, one or more processor cores, a digital signal processor, a programmable gate array, a microcontroller, an application specific integrated circuit "ASIC", etc.), a semiconductor memory, or combinatorial elements.

Additionally, or in the alternative, the term logic may refer to or include software such as one or more processes, one or more instances, Application Programming Interface(s) (API), subroutine(s), function(s), applet(s), servlet(s), routine(s), source code, object code, shared library/dynamic link library (dll), or even one or more instructions. This software may be stored in any type of a suitable non-transitory storage medium, or transitory storage medium (e.g., electrical, optical, acoustical or other form of propagated signals such as carrier waves, infrared signals, or digital signals). Examples of a non-transitory storage medium may include, but are not limited or restricted to a programmable circuit; non-persistent storage such as volatile memory (e.g., any type of random access memory "RAM"); or persistent storage such as non-volatile memory (e.g., read-only memory "ROM", power-backed RAM, flash memory, phase-change memory, etc.), a solid-state drive, hard disk drive, an optical disc drive, or a portable memory device. As firmware, the logic may be stored in persistent storage.

With respect to "alternative reality," the term alternative reality may pertain to virtual reality, augmented reality, and mixed reality unless context suggests otherwise. "Virtual reality" includes virtual content in a virtual setting, which setting can be a fantasy or a real-world simulation. "Augmented reality" and "mixed reality" include virtual content in a real-world setting such as a real depiction of a portion of a patient's body including the anatomical element. Augmented reality includes the virtual content in the real-world setting, but the virtual content is not necessarily anchored in the real-world setting. For example, the virtual content can be information overlying the real-world setting. The information can change as the real-world setting changes due to time or environmental conditions in the real-world setting, or the information can change as a result of a consumer of the augmented reality moving through the real-world setting; however, the information remains overlying the real-world setting. Mixed reality includes the virtual content anchored in every dimension of the real-world setting. For example, the virtual content can be a virtual object anchored in the real-world setting. The virtual object can change as the real-world setting changes due to time or environmental conditions in the real-world setting, or the virtual object can change to accommodate the perspective of a consumer of the mixed reality as the consumer moves through the real-world setting. The virtual object can also change in accordance with any interactions with the consumer or another real-world or virtual agent. Unless the virtual object is moved to another location in the real-world setting by the consumer of the mixed reality, or some other real world or virtual agent, the virtual object remains anchored in the real-world setting. Mixed reality does not exclude the foregoing information overlying the real-world setting described in reference to augmented reality.

Unless defined otherwise, all technical and scientific terms used herein have the same meaning as commonly understood by those of ordinary skill in the art.

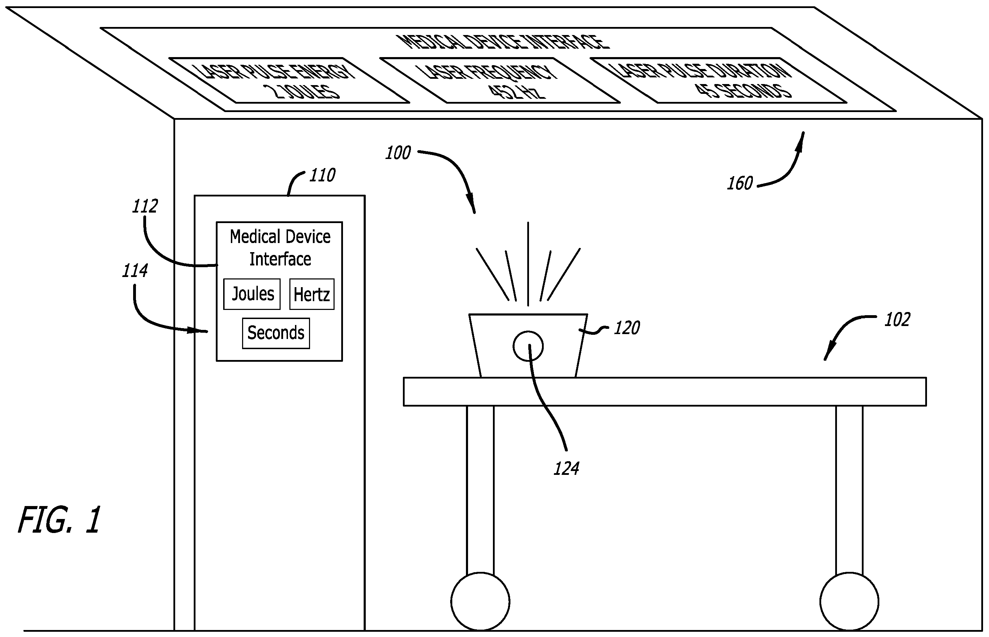

FIG. 1 illustrates a perspective view of a medical device projection system 100, in accordance with some embodiments. In some embodiments, the medical device projection system 100 includes a first medical device 110, residing outside a sterile field 102. In some embodiments, the first medical device 110 includes a screen 112, depicting a user control interface 114 including a plurality of parameters of the first medical device 110. In some embodiments, the first medical device 110 includes a laser that may be configured to dissolve a kidney stone. In some embodiments, the plurality of parameters may include laser pulse energy, laser frequency, laser pulse duration, average power, pulse width, or medical device status (e.g., a standby mode, an active mode or the like). The medical device projection system 100 further includes a projector 120 within the sterile field 102, in communication with the first medical device 110. In some embodiments, the projector 120 may be sterile, configured to be sterilized, or placed within a sterile bag. In some embodiments, the projector 120 may be sufficiently robust to remain functional following sterilization cycle or constructed from materials that can withstand typical sterilize techniques (e.g., hardened plastics, polymers or the like).

In some embodiments, the projector 120 may be wired to the first medical device 110 or in wireless communication with the first medical device 110. Exemplary wireless communication modalities can include WiFi, Bluetooth, Near Field Communications (NFC), cellular Global System for Mobile Communication ("GSM"), electromagnetic (EM), radio frequency (RF), combinations thereof, or the like. In some embodiments, the projector 120 may be configured to project a mixed reality visual representation of the user control interface 114 on a surface 160 visible to the user. The depiction of the user control interface 114 on the surface 160 visible to the user allows the user to visualize the user control interface 114 including the plurality of parameters, confirming desired parameters or the need to change existing parameters, all in real time. In some embodiments, the surface 160 may be within the sterile field 102 or outside of the sterile field 102. In some embodiments, the surface 160 may include a ceiling, a floor, a wall, a surface of a second medical device, a sterile drape or the like.

In some embodiments, the projector 120 may be configured to not only project a mixed reality depiction of the user control interface 114 but also control one or more of the plurality of parameters. The projector 120 is configured to include one or more buttons 124 configured to control the plurality of parameters of the first medical device 110. In some embodiments, the one or more buttons 124 may be configured to be manipulated by a user. In some embodiments, the one or more buttons 124 may include a directional pad or a clickable scroll wheel configured to allow a user to select a parameter using the click function and then change the parameter using the scroll function. Advantageously, the medical device projection system 100 allows the projector 120 to reside within the sterile field 102 while projecting a mixed reality visual representation of the user control interface 114 of the first medical device 110 onto a surface 160 visible to the user and provide parameter changes to the first medical device 110 from within the sterile field 102.

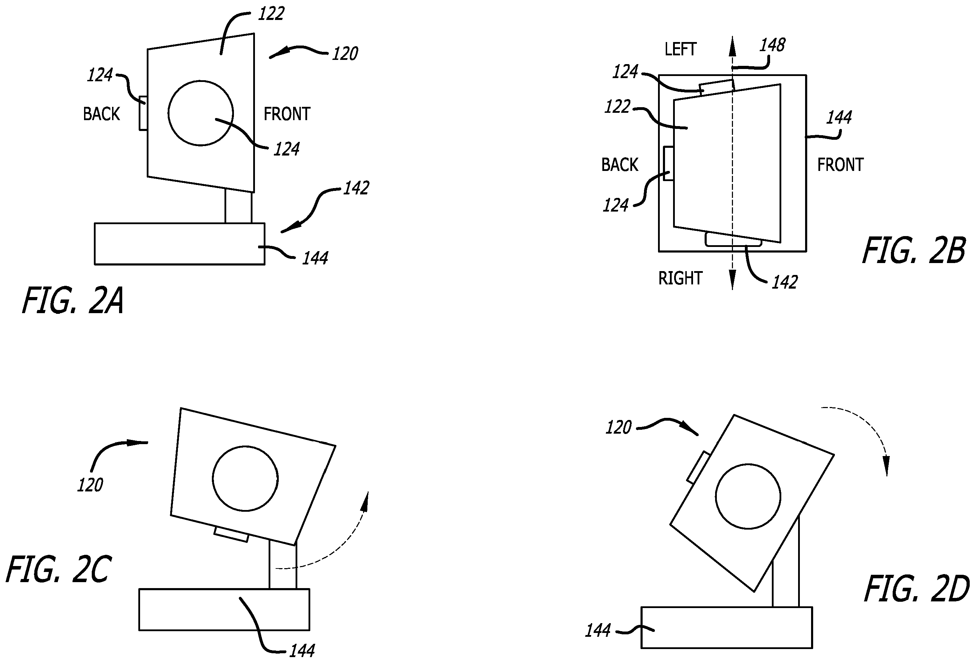

FIG. 2A illustrates a cross sectional side view of the projector 120, in accordance with some embodiments. In some embodiments, the projector 120 includes a projector body 122 having a front side and a back side. In some embodiments, the projector body 122 may include the one or more buttons 124 thereon. In some embodiments, the projector body 122 may be coupled to a projector stand 142 configured to stabilize the projector 120 when the projector 120 is within the sterile field 102. In some embodiments, the projector stand 142 may include a projector stand base 144 or two or more projector stand legs. The projector stand 142 may be configured to allow the projector 120 to rotate annularly around a projector stand axis 148, as will be described in more detail herein.

FIG. 2B illustrates a plan view of the projector 120, in accordance with some embodiments. The projector body 122 may have a left side or a right side. The one or more buttons 124 are coupled to the left side, the right side, the back side, or a combination thereof. In some embodiments, the projector stand 142 may be coupled to the left side or the right side. In some embodiments, the one or more buttons 124 may be coupled to one side and the projector stand 142 may be coupled to the opposite side, allowing the user to adjust projection using the projector stand 142 and adjust the one or more parameters using the one or more buttons 124. In some embodiments, where the projector stand 142 couples to the projector 120 defines the projector stand axis 148.

FIGS. 2C-2D illustrate a cross sectional view of an exemplary method of adjusting the projector 120 to project onto a variety of surfaces 160, in accordance with some embodiments. The projector 120 may be rotated around the projector stand axis 148 (see FIG. 2B) to configure the projector 120 to project the mixed reality visual representation of the user control interface 114 on a variety of surfaces 160 (see FIG. 1). In some embodiments, the projector 120 may be rotated upward to be perpendicular to the projector stand base 144. In some embodiments, the projector 120 may rotate upward through an angle of 0-110° in relation to the projector stand base 144 as illustrated in FIG. 2C and may rotate downward through an angle of 0 to -60° in relation to the projector stand base 144 as illustrated in FIG. 2D. Rotating the projector 120 upwards through an angle of 0-110° allows the projector 120 to project on a wall surface or ceiling surface. Rotating the projector 120 downward through an angle of 0 to -60° allows the projector to project on a sterile drape or a surface of a second medical device. Advantageously, having the projector 120 configured to rotate upward or downward to project on a surface 160 allows a user to operate within the sterile field 102 while visualizing or controlling the user control interface 114 from within the sterile field 102.

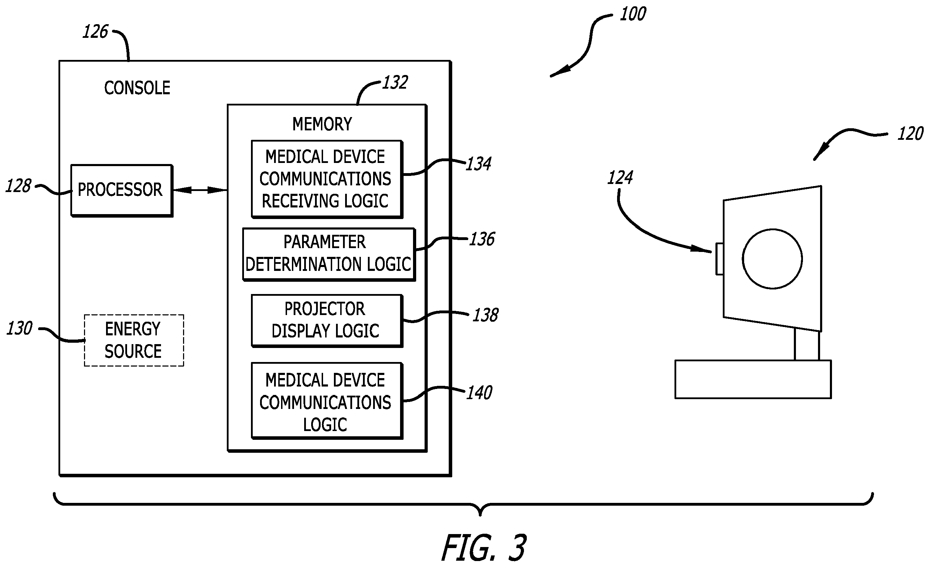

FIG. 3 illustrates a block diagram of some components of the medical device projection system 100 including a projector console 126, in accordance with some embodiments. In some embodiments, the projector 120 may be configured to include a projector console 126 including one or more processors 128, an energy source 130, non-transitory computer readable medium ("memory") 132 and a plurality of logic modules. In some embodiments, the projector console 126 may be coupled to the projector 120, located within the projector 120, or located within the projector stand 142. In some embodiments, the plurality of logic modules may be configured to include one or more of: a medical device communications receiving logic 134, a parameter determination logic 136, a projector display logic 138, and a medical device communications logic 140. In some embodiments, the medical device communications receiving logic 134 may be configured to receive communications from the first medical device 110 including the depiction of the user control interface 114. In some embodiments, the parameter determination logic 136 may be configured to correlate the status (e.g., physical location, orientation, or the like) of the one or more buttons 124 with one or more parameter changes on the user control interface 114. In some embodiments, the projector display logic 138 may be configured to project the mixed reality depiction of the user control interface 114, including any parameter changes, on the surface 160. In some embodiments, the medical device communications logic 140 may be configured to transmit the one or more parameter changes from the projector 120 to the first medical device 110.

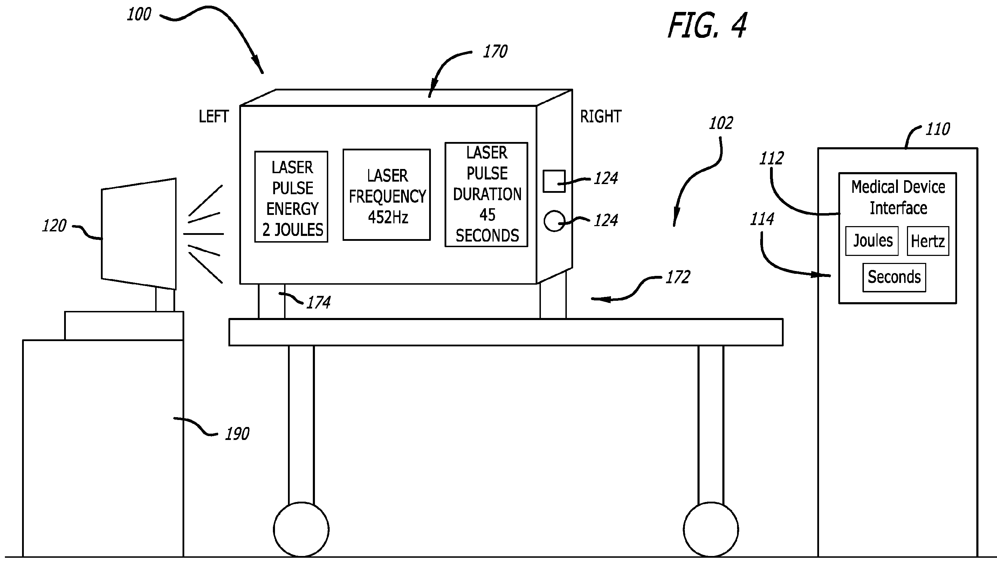

FIG. 4 illustrates a perspective view of the medical device projection system 100, in accordance with some embodiments. In some embodiments, the medical device projection system 100 includes the first medical device 110 as described above. In some embodiments, the medical device projection system 100 includes the projector 120 as described above configured to project a mixed reality visual representation of the user control interface 114 on a display screen 170 that is visible to a user. In some embodiments, the projector 120 may reside outside the sterile field 102 and the display screen 170 may reside within the sterile field 102. In some embodiments, the projector 120 may reside within the sterile field 102. In some embodiments, the display screen 170 may be sterile, configured to be sterilized, or placed within a sterile bag. In some embodiments, the display screen 170 may be sufficiently robust to remain functional following sterilization cycle or constructed from materials that can withstand typical sterilize techniques (e.g., hardened plastics, polymers or the like).

In some embodiments, the projector 120 may be in wireless communication with the display screen 170. Exemplary wireless communication modalities can include WiFi, Bluetooth, Near Field Communications (NFC), cellular Global System for Mobile Communication ("GSM"), electromagnetic (EM), radio frequency (RF), combinations thereof, or the like. In some embodiments, the projector 120 may be configured to display on the display screen 170 a mixed reality visual depiction of the user control interface 114. In some embodiments, the display screen 170 may include a heads up display, allowing the user to see the mixed reality visual representation of the user control interface 114 on the display screen 170, but also see real world objects through the display screen 170 including the patient. In some embodiments, the display screen 170 may be curved to allow for optimal user viewing from all angles. In some embodiments, the display screen 170 may include one or more buttons 124 configured to control parameters on the user control interface 114. In some embodiments, the one or more buttons 124 may be manipulated by a user. In some embodiments, the one or more buttons 124 may be coupled to a right side or a left side of the display screen 170.

In some embodiments, the display screen 170 may include a display screen base 172, having two or more display screen legs 174 configured to stabilize the display screen 170 within the sterile field 102. In some embodiments, the projector 120 may be configured to be coupled to the first medical device 110 or may be coupled to a second medical device 190. In some embodiments, the second medical device 190 may be outside of the sterile field 102 or within the sterile field 102. In an embodiment, the projector 120 may be contained within a dongle configured to be plugged into a computing device or the first medical device 110 or the second medical device. In this embodiment, the computing device, first medical device 110 or the second medical device 190 may be configured to provide an energy source for the projector 120.

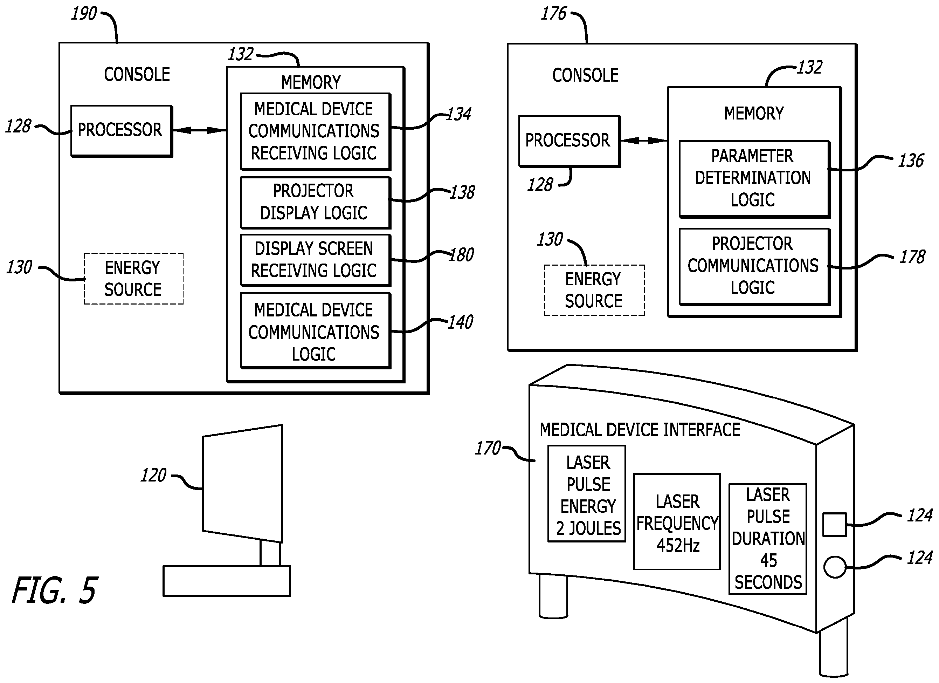

FIG. 5 illustrates a block diagram of some components of the medical device projection system 100 including the projector 120, the projector console 126, and a display screen console 176, in accordance with some embodiments. In some embodiments, the projector 120 may include the projector console 126 and the display screen 170 may include a display screen console 176. In some embodiments, the projector console 126 includes one or more processors 128, a first energy source 130, non-transitory computer readable medium ("memory") 132 and a first plurality of logic modules. In some embodiments, the plurality of logic modules may include one or more of the medical device communications receiving logic 134, the projector display logic 138 and the medical device communications logic 140, as described above.

In some embodiments, the plurality of logic modules may include a display screen receiving logic 180. In some embodiments, the display screen receiving logic 180 may be configured to receiving the one or more parameter changes from the display screen 170. In some embodiments, the display screen console 176 may include one or more processors 128, a second energy source 130, non-transitory computer readable medium ("memory") 132 having two or more logic modules. In some embodiments, the two or more logic modules may include the parameter determination logic 136 or a projector communications logic 178. In some embodiments, the parameter determination logic 136 may be configured to correlate the status of the one or more buttons 124 with one or more parameter changes on the user control interface 114. In some embodiments, the projector communications logic 178 may be configured to transmit the one or more parameter changes to the projector 120.

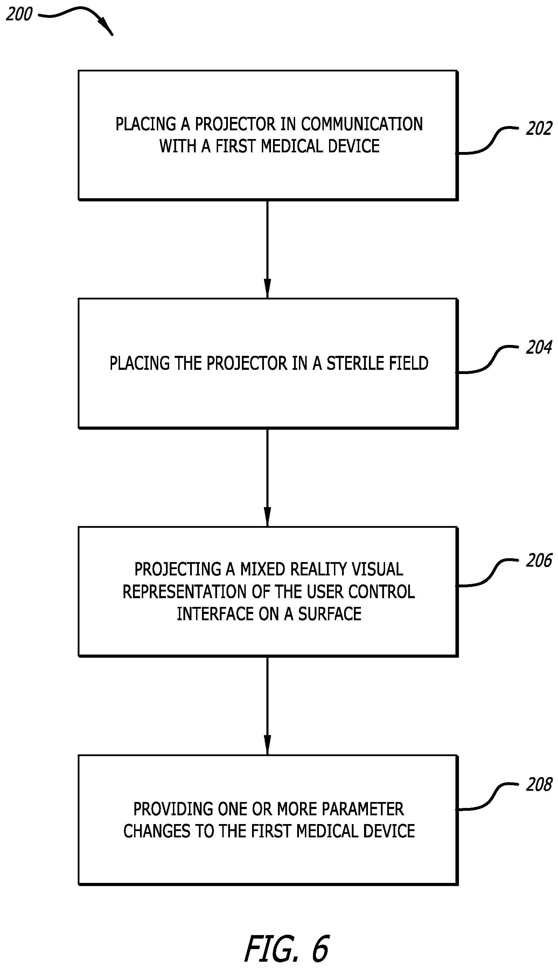

FIG. 6 illustrates a flow chart of an exemplary method 200 of controlling a plurality of parameter changes of a first medical device 110 from within the sterile field 102, in accordance with some embodiments. In some embodiments, the method 200 includes placing the projector 120 in communication with the first medical device 110 (block 202). In some embodiments, the first medical device 110 is outside of the sterile field 102 and includes the user control interface 114 having the plurality of parameters. In some embodiments, the projector 120 includes the one or more buttons 124 configured to control the plurality of parameters. In some embodiments, the one or more buttons 124 may include a clickable scroll wheel. In some embodiments, placing the projector 120 in communication with the first medical device 110 includes placing the projector 120 in wireless communication with the first medical device 110.

The method 200 further includes placing the projector 120 in the sterile field 102 (block 204). In some embodiments, the projector 120 is sterile. In some embodiments, placing the projector 120 in the sterile field 102 includes placing the projector 120 in a sterile bag. In some embodiments, the sterile bag is clear. The method 200 further includes projecting a mixed reality visual representation of the user control interface 114 on a surface 160 (block 206). In some embodiments, projecting a mixed reality visual representation of the user control interface 114 includes projecting a mixed reality visual representation of the user control interface 114 as the user provides parameter changes in real time. In some embodiments, the surface 160 is visible to a user. In some embodiments, the surface 160 includes a floor, a ceiling, a surface of a second medical device, a sterile drape or the like.

The method 200 further includes providing one or more parameter changes to the first medical device 110 (block 208). In some embodiments, providing one or more parameter changes to the first medical device 110 includes the one or more buttons 124 being manipulated by the user to provide the one or more parameter changes to the first medical device 110. For example, when the one or more buttons 124 include the clickable scroll wheel, the user may twist the clickable scroll wheel to increase or decrease one or more parameters. In some embodiments, providing one or more parameter changes include the projector 120 transmitting the one or more parameter changes to the first medical device 110. In some embodiments, the first medical device 110 may include a laser. In some embodiments, the plurality of parameters may include laser pulse energy, laser frequency and laser pulse duration.

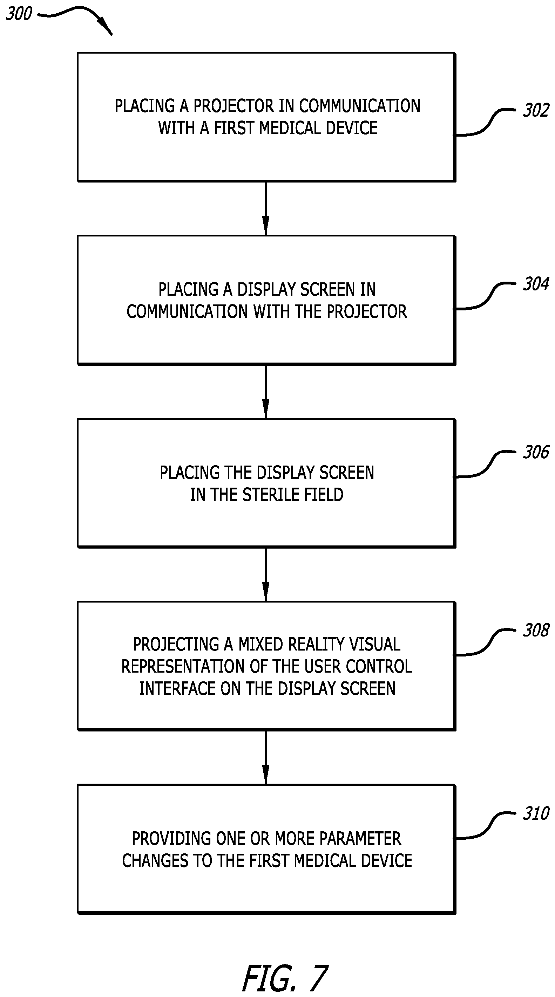

FIG. 7 illustrates a flow chart of an exemplary method 300 of controlling a plurality of parameter changes of a first medical device 110 from within a sterile field 102, in accordance with some embodiments. The method 300 includes placing the projector 120 in communication with the first medical device 110 of the medical device projection system 100 (block 302). In some embodiments, placing the projector 120 in communication with the first medical device 110 includes placing the projector120 in wireless communication with the first medical device 110. In some embodiments, placing the projector 120 in communication with the first medical device 110 includes placing the projector 120 in communication with the first medical device 110 having the user control interface 114 with the plurality of parameters. In some embodiments, placing the projector 120 in communication with the first medical device 110 includes placing the projector 120 in communication with the first medical device 110, wherein the first medical device 110 may be outside of the sterile field 102.

The method 300 includes placing the display screen 170 in communication with the projector 120 (block 304). In some embodiments, placing the display screen 170 in communication with the projector 120 includes placing the display screen 170 in wireless communication with the projector 120. In some embodiments, the display screen 170 includes one or more buttons 124 configured to control the plurality of parameters. In some embodiments, the one or more buttons 124 include a clickable scroll wheel. The method 300 further includes placing the display screen 170 in the sterile field 102 (block 306). In some embodiments, the display screen 170 is visible to a user. In some embodiments, the display screen 170 is sterile. In some embodiments, the display screen 170 may be placed in a sterile bag. In some embodiments, the sterile bag is clear. The method 300 further includes projecting a mixed reality visual representation of the user control interface 114 on the display screen 170 (block 308). In some embodiments, projecting a mixed reality visual representation of the user control interface 114 includes projecting a mixed reality visual representation of the user control interface 114 as the user provides parameter changes in real time.

The method 300 further includes providing one or more parameter changes to the first medical device 110 (block 310). In some embodiments, providing one or more parameter changes include the one or more buttons 124 on the display screen 170 being manipulated by the user to provide the changes. For example, when the one or more buttons 124 include the clickable scroll wheel, the user may twist the clickable scroll wheel to increase or decrease one or more parameters. In some embodiments, providing one or more changes include the display screen 170 transmitting the one or more parameter changes to the projector 120 and the projector 120 transmitting the one or more parameter changes to the first medical device 110. In some embodiments, the first medical device 110 may include a laser. In some embodiments, the plurality of parameters may include laser pulse energy, laser frequency and laser pulse duration.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A medical device projection system (100), comprising:
a first medical device (110) configured to be placed outside of a sterile field (102), the first medical device (110) having a user control interface (114) including a plurality of parameters; and
a projector (120) configured to be placed within the sterile field (102), the projector (120) in communication with the first medical device (110) and configured to project a visual representation of the user control interface (114) on a surface (160) and control one or more of the plurality of parameters,
wherein the projector (120) includes one or more buttons (124) configured to control the plurality of parameters, the one or more buttons (124) being coupled to a left side, a right side, or a back side of a projector body (122).

2. The medical device projection system (100) according to claim 1, wherein the surface (160) is visible to a user and is configured to be placed inside the sterile field (102) or outside the sterile field (102).

3. The medical device projection system (100) according to claim 2, wherein the surface (160) includes a floor, a ceiling, a surface of a second medical device, or a sterile drape.

4. The medical device projection system (100) according to any of the preceding claims, wherein the one or more buttons (124) include a clickable scroll wheel.

5. The medical device projection system (100) according to any of the preceding claims, wherein the projector (120) includes a projector body (122) coupled to a projector stand (142), the projector stand (142) coupled to a right side or a left side of the projector body (122).

6. The medical device projection system (100) according to claim 5, wherein the projector stand (142) includes a projector stand base (144) or two or more projector stand legs.

7. The medical device projection system (100) according to claim 5, wherein the projector (120) is configured to rotate annularly around a projector stand axis.

8. The medical device projection system (100) according to any of the preceding claims, wherein the projector (120) includes a console (126) having an energy source (130), one or more processors (128), non-transitory computer readable medium (132) and a plurality of logic modules.

9. The medical device projection system (100) according to claim 8, wherein the plurality of logic modules, when executed by the processor (128), are configured to perform operations including:
receiving a depiction of the user control interface (114) from the first medical device (110);
correlating the status of the one or more buttons (124) with one or more parameter changes on the user control interface (114);
projecting a visual representation of the user control interface (114), including any parameter changes on a surface (160); and
transmitting the one or more parameter changes from the projector (120) to the first medical device (110).

10. The medical device projection system (100) according to any of the preceding claims, wherein the first medical device (110) includes a laser and the plurality of parameters include laser pulse energy, laser frequency, and laser pulse duration, and/or wherein the projector (120) is configured to be sterile or placed into a sterile bag.

11. A method of controlling a plurality of parameter changes of a medical device from within a sterile field (102), comprising
placing a projector (120), having one or more buttons (124), in communication with a first medical device (110), the first medical device (110) having a user control interface (114) with a plurality of parameters, the first medical device (110) being outside of a sterile field (102), wherein the one or more buttons (124) are configured to control the plurality of parameters;
placing the projector (120) in the sterile field (102);
projecting a visual representation of the user control interface (114) on a surface (160); and
providing one or more parameter changes to the first medical device (110).

12. The method according to claim 11, wherein placing the projector (120) having the one or more buttons (124) in communication with the first medical device (110) includes placing the projector (120) having the one or more buttons (124) configured to control the plurality of parameters, in communication with the first medical device (110), and/or wherein placing the projector (120) in the sterile field (102) includes placing the projector (120) in a sterile bag.

13. The method according to either claim 11 or claim 12, wherein projecting the visual representation of the user control interface (114) on the surface (160) includes projecting the visual representation of the user control interface (114) on the surface (160) wherein the surface (160) is visible to a user and includes a floor, a ceiling, a surface of a second medical device, or a sterile drape.

14. The method according to either claim 11 or claim 12, wherein providing one or more parameter changes to the first medical device (110) include providing one or more parameter changes to the first medical device (110) wherein the first medical device (110) includes a laser and the one or more parameter changes include laser pulse energy, laser frequency or laser pulse duration.

## Patentansprüche

1. Projektionssystem für medizinische Vorrichtung (100), umfassend:
eine erste medizinische Vorrichtung (110), die dazu ausgebildet ist, außerhalb eines sterilen Feldes (102) angeordnet zu werden, wobei die erste medizinische Vorrichtung (110) eine Benutzersteuerschnittstelle (114) einschließlich einer Vielzahl von Parametern aufweist; und
einen Projektor (120), der dazu ausgebildet ist, innerhalb des sterilen Feldes (102) angeordnet zu werden, wobei der Projektor (120) mit der ersten medizinischen Vorrichtung (110) in Kommunikation steht und dazu ausgebildet ist, eine visuelle Darstellung der Benutzersteuerschnittstelle (114) auf eine Oberfläche (160) zu projizieren und einen oder mehrere der Vielzahl von Parametern zu steuern,
wobei der Projektor (120) eine oder mehrere Tasten (124) einschließt, die dazu ausgebildet sind, die Vielzahl von Parametern zu steuern, wobei die eine oder die mehreren Tasten (124) mit einer linken Seite, einer rechten Seite oder einer Rückseite eines Projektorkörpers (122) gekoppelt sind.

2. Projektionssystem für medizinische Vorrichtung (100) nach Anspruch 1, wobei die Oberfläche (160) für einen Benutzer sichtbar ist und dazu ausgebildet ist, innerhalb des sterilen Feldes (102) oder außerhalb des sterilen Feldes (102) angeordnet zu sein.

3. Projektionssystem für medizinische Vorrichtung (100) nach Anspruch 2, wobei die Oberfläche (160) einen Boden, eine Decke, eine Oberfläche einer zweiten medizinischen Vorrichtung oder ein steriles Abdecktuch einschließt.

4. Projektionssystem für medizinische Vorrichtung (100) nach einem der vorstehenden Ansprüche, wobei die eine oder mehreren Tasten (124) ein drückbares Scrollrad einschließen.

5. Projektionssystem für medizinische Vorrichtung (100) nach einem der vorstehenden Ansprüche, wobei der Projektor (120) einen Projektorkörper (122) einschließt, der mit einem Projektorständer (142) gekoppelt ist, wobei der Projektorständer (142) mit einer rechten Seite oder einer linken Seite des Projektorkörpers (122) gekoppelt ist.

6. Projektionssystem für medizinische Vorrichtung (100) nach Anspruch 5, wobei der Projektorständer (142) eine Basis des Projektorständers (144) oder zwei oder mehr Beine des Projektorständers einschließt.

7. Projektionssystem für medizinische Vorrichtung (100) nach Anspruch 5, wobei der Projektor (120) dazu ausgebildet ist, sich kreisförmig um eine Projektorständerachse zu drehen.

8. Projektionssystem für medizinische Vorrichtung (100) nach einem der vorstehenden Ansprüche, wobei der Projektor (120) eine Konsole (126) einschließt, die eine Energiequelle (130), einen oder mehrere Prozessoren (128), ein nicht-transitorisches computerlesbares Medium (132) und eine Vielzahl von Logikmodulen aufweist.

9. Projektionssystem für medizinische Vorrichtung (100) nach Anspruch 8, wobei die Vielzahl von Logikmodulen, wenn sie durch den Prozessor (128) ausgeführt werden, dazu ausgebildet sind, Operationen auszuführen, einschließend:
Empfangen einer Abbildung der Benutzersteuerschnittstelle (114) von der ersten medizinischen Vorrichtung (110);
Korrelieren des Status der einen oder der mehreren Tasten (124) mit einer oder mehreren Parameteränderungen auf der Benutzersteuerschnittstelle (114);
Projizieren einer visuellen Darstellung der Benutzersteuerschnittstelle (114), einschließlich jeder Parameteränderung, auf eine Oberfläche (160); und
Übertragen der einen oder der mehreren Parameteränderungen von dem Projektor (120) an die erste medizinische Vorrichtung (110).

10. Projektionssystem für medizinische Vorrichtung (100) nach einem der vorstehenden Ansprüche, wobei die erste medizinische Vorrichtung (110) einen Laser einschließt und die Vielzahl von Parametern Laserpulsenergie, Laserfrequenz und Laserpulsdauer einschließt, und/oder wobei der Projektor (120) dazu ausgebildet ist, steril zu sein oder in einen sterilen Beutel eingebracht zu werden.

11. Verfahren zum Steuern einer Vielzahl von Parameteränderungen einer medizinischen Vorrichtung von innerhalb eines sterilen Feldes (102), umfassend
Anordnen eines Projektors (120), der eine oder mehrere Tasten (124) aufweist, in Kommunikation mit einer ersten medizinischen Vorrichtung (110), wobei die erste medizinische Vorrichtung (110) eine Benutzersteuerschnittstelle (114) mit einer Vielzahl von Parametern aufweist, wobei die erste medizinische Vorrichtung (110) außerhalb eines sterilen Feldes (102) ist, wobei die eine oder mehreren Tasten (124) dazu ausgebildet sind, die Vielzahl von Parametern zu steuern;
Anordnen des Projektors (120) in dem sterilen Feld (102);
Projizieren einer visuellen Darstellung der Benutzersteuerschnittstelle (114) auf eine Oberfläche (160); und
Bereitstellen einer oder mehrerer Parameteränderungen an die erste medizinische Vorrichtung (110).

12. Verfahren nach Anspruch 11, wobei das Anordnen des Projektors (120), der die eine oder die mehreren Tasten (124) in Kommunikation mit der ersten medizinischen Vorrichtung (110) aufweist, das Anordnen des Projektors (120), der die eine oder die mehreren Tasten (124) aufweist, die dazu ausgebildet sind, die Vielzahl von Parametern zu steuern, in Kommunikation mit der ersten medizinischen Vorrichtung (110) einschließt, und/oder wobei das Anordnen des Projektors (120) in dem sterilen Feld (102) das Anordnen des Projektors (120) in einem sterilen Beutel einschließt.

13. Verfahren nach entweder Anspruch 11 oder Anspruch 12, wobei das Projizieren der visuellen Darstellung der Benutzersteuerschnittstelle (114) auf die Oberfläche (160) das Projizieren der visuellen Darstellung der Benutzersteuerschnittstelle (114) auf die Oberfläche (160) einschließt, wobei die Oberfläche (160) für einen Benutzer sichtbar ist und einen Boden, eine Decke, eine Oberfläche einer zweiten medizinischen Vorrichtung oder ein steriles Abdecktuch einschließt.

14. Verfahren nach entweder Anspruch 11 oder Anspruch 12, wobei das Bereitstellen einer oder mehrerer Parameteränderungen an die erste medizinische Vorrichtung (110) das Bereitstellen einer oder mehrerer Parameteränderungen an die erste medizinische Vorrichtung (110) einschließt, wobei die erste medizinische Vorrichtung (110) einen Laser einschließt und die eine oder mehreren Parameteränderungen Laserpulsenergie, Laserfrequenz oder Laserpulsdauer einschließen.

## Revendications

1. Système de projection de dispositif médical (100), comprenant :
un premier dispositif médical (110) configuré pour être placé à l'extérieur d'un champ stérile (102), le premier dispositif médical (110) présentant une interface de commande utilisateur (114) incluant une pluralité de paramètres ; et
un projecteur (120) configuré pour être placé dans le champ stérile (102), le projecteur (120) étant en communication avec le premier dispositif médical (110) et configuré pour projeter une représentation visuelle de l'interface de commande utilisateur (114) sur une surface (160) et commander un ou plusieurs paramètres parmi la pluralité de paramètres,
dans lequel le projecteur (120) inclut un ou plusieurs boutons (124) configurés pour commander la pluralité de paramètres, les un ou plusieurs boutons (124) étant couplés à un côté gauche, un côté droit, ou un côté arrière d'un corps de projecteur (122).

2. Système de projection de dispositif médical (100) selon la revendication 1, dans lequel la surface (160) est visible pour un utilisateur et est configurée pour être placée à l'intérieur du champ stérile (102) ou à l'extérieur du champ stérile (102).

3. Système de projection de dispositif médical (100) selon la revendication 2, dans lequel la surface (160) inclut un sol, un plafond, une surface d'un deuxième dispositif médical, ou un champ opératoire stérile.

4. Système de projection de dispositif médical (100) selon l'une quelconque des revendications précédentes, dans lequel les un ou plusieurs boutons (124) incluent une molette de défilement cliquable.

5. Système de projection de dispositif médical (100) selon l'une quelconque des revendications précédentes, dans lequel le projecteur (120) inclut un corps de projecteur (122) couplé à un support de projecteur (142), le support de projecteur (142) étant couplé à un côté droit ou un côté gauche du corps de projecteur (122).

6. Système de projection de dispositif médical (100) selon la revendication 5, dans lequel le support de projecteur (142) inclut une base de support de projecteur (144) ou deux ou plusieurs pieds de support de projecteur.

7. Système de projection de dispositif médical (100) selon la revendication 5, dans lequel le projecteur (120) est configuré pour tourner annulairement autour d'un axe de support de projecteur.

8. Système de projection de dispositif médical (100) selon l'une quelconque des revendications précédentes, dans lequel le projecteur (120) inclut une console (126) présentant une source d'énergie (130), un ou plusieurs processeurs (128), un support lisible par ordinateur non transitoire (132) et une pluralité de modules logiques.

9. Système de projection de dispositif médical (100) selon la revendication 8, dans lequel la pluralité de modules logiques, lorsqu'ils sont exécutés par le processeur (128), sont configurés pour effectuer des opérations incluant :
recevoir une représentation de l'interface de commande utilisateur (114) depuis le premier dispositif médical (110) ;
corréler l'état des un ou plusieurs boutons (124) avec une ou plusieurs modifications de paramètres sur l'interface de commande utilisateur (114) ;
projeter une représentation visuelle de l'interface de commande utilisateur (114), incluant toutes modifications de paramètres sur une surface (160) ; et
transmettre les une ou plusieurs modifications de paramètres depuis le projecteur (120) vers le premier dispositif médical (110).

10. Système de projection de dispositif médical (100) selon l'une quelconque des revendications précédentes, dans lequel le premier dispositif médical (110) inclut un laser et la pluralité de paramètres inclut une énergie d'impulsion laser, une fréquence laser, et une durée d'impulsion laser, et/ou dans lequel le projecteur (120) est configuré pour être stérile ou placé dans un sac stérile.

11. Procédé de commande d'une pluralité de modifications de paramètres d'un dispositif médical depuis l'intérieur d'un champ stérile (102), comprenant
placer un projecteur (120), présentant un ou plusieurs boutons (124), en communication avec un premier dispositif médical (110), le premier dispositif médical (110) présentant une interface de commande utilisateur (114) avec une pluralité de paramètres, le premier dispositif médical (110) étant à l'extérieur d'un champ stérile (102), dans lequel les un ou plusieurs boutons (124) sont configurés pour commander la pluralité de paramètres ;
placer le projecteur (120) dans le champ stérile (102) ;
projeter une représentation visuelle de l'interface de commande utilisateur (114) sur une surface (160) ; et
fournir une ou plusieurs modifications de paramètres au premier dispositif médical (110).

12. Procédé selon la revendication 11, dans lequel l'emplacement du projecteur (120) présentant les un ou plusieurs boutons (124) en communication avec le premier dispositif médical (110) inclut l'emplacement du projecteur (120) présentant les un ou plusieurs boutons (124) configurés pour commander la pluralité de paramètres, en communication avec le premier dispositif médical (110), et/ou dans lequel l'emplacement du projecteur (120) dans le champ stérile (102) inclut le placement du projecteur (120) dans un sac stérile.

13. Procédé selon la revendication 11 ou la revendication 12, dans lequel la projection de la représentation visuelle de l'interface de commande utilisateur (114) sur la surface (160) inclut la projection de la représentation visuelle de l'interface de commande utilisateur (114) sur la surface (160), dans lequel la surface (160) est visible pour un utilisateur et inclut un sol, un plafond, une surface d'un deuxième dispositif médical, ou un champ opératoire stérile.

14. Procédé selon la revendication 11 ou la revendication 12, dans lequel la fourniture d'une ou plusieurs modifications de paramètres au premier dispositif médical (110) inclut la fourniture d'une ou plusieurs modifications de paramètres au premier dispositif médical (110) dans lequel le premier dispositif médical (110) inclut un laser et les une ou plusieurs modifications de paramètres incluent une énergie d'impulsion laser, une fréquence laser ou une durée d'impulsion laser.
